# EUROPÄISCHE PATENTSCHRIFT

(11) **EP 1 330 225 B1**
(45) Veröffentlichungstag und Bekanntmachung des Hinweises auf die Patenterteilung: **15.09.2004**
(21) Anmeldenummer: 01992544.5
(22) Anmeldetag: 31.10.2001
(51) Int. Cl.: A61F 13/53

(54) **ABSORBIERENDER ARTIKEL MIT FLÜSSIGKEITSVERTEILUNGSSCHICHT UND GEZIELTEM FLÜSSIGKEITSTRANSFER BEI DRUCKEINWIRKUNG**
ABSORBENT ARTICLE COMPRISING A FLUID DISTRIBUTION LAYER AND A TARGETED FLUID TRANSFER INDUCED BY PRESSURE
ARTICLE ABSORBANT CONTENANT UNE COUCHE DE REPARTITION DES LIQUIDES ET A TRANSFERT CIBLE DE LIQUIDES SOUS L'ACTION D'UNE PRESSION

(30) Priorität: 31.10.2000 DE 10053895
(43) Veröffentlichungstag der Anmeldung: 30.07.2003
(73) Patentinhaber: Corovin GmbH, 31224 Peine (DE)
(72) Erfinder: ROETTGER, Henning, 38108 Braunschweig (DE); BAUER, Joachim, 5445 Eggenwil (CH); ROY, Indra, 5445 Eggenwil (CH)
(86) Internationale Anmeldenummer: PCT/EP2001/012626
(87) Internationale Veröffentlichungsnummer: WO 2002/036058

(56) Entgegenhaltungen:
- EP-A- 0 536 941
- WO-A-00/59439
- WO-A-01/26596
- WO-A-95/17870
- WO-A-99/23285
- DE-A- 3 525 379
- DE-B- 2 208 126
- DE-C- 19 915 716

## Beschreibung

Die vorliegende Erfindung betrifft ein flüssigkeitsaufnehmendes Wegwerfprodukt, beispielsweise ein Einmalprodukt, insbesondere ein Hygieneprodukt. Das Produkt hat eine Oberlage und einen flüssigkeitsaufnehmenden und -speichernden Kern.

Aus dem Stand der Technik ist das Problem bekannt, Flüssigkeit in einem Hygieneprodukt zurückhalten zu können.

Aus DE 2 208 126 geht ein umhülltes Saugkissen für hygienische Zwecke hervor, dessen Saugschicht wenigstens eine Schicht aus Quellstoffteilchen in Granulatform aufweist, die auf einem Träger fixiert sind, wobei der Träger mit den fixierten Quellstoffteilchen in einer der dem Körper zugewandten oben liegenden Schichten des Saugkörpers angeordnet ist und bei einer Druckeinwirkung eine Rücknässung der Kissen vermieden wird.

In WO 01/26596 wird ein Absorberartikel mit einem flüssigkeitsundurchlässigen Backsheet, einem flüssigkeitsdurchlässigen Topsheet, einem Absorbersystem beschrieben, welches zwischen dem Backsheet und dem Topsheet angeordnet ist und ein erstes und zweites Absorbercomposit mit unterschiedlichen Permeabilitäten, Kapillaritäten, Porositäten, Dicken und Aufnahmevermögen aufweist, wobei die Absorbersysteme in Fluidverbindung stehen.

Aus DE 199 15 716 ist ein absorbierender Einwegartikel mit geringer Neigung zur Rücknässung bekannt, welcher aus einer oberen, dem Körper zugewandten flüssigkeitsdurchlässigen Abdeckschicht, aus einer unteren, der Bekleidung des Trägers zugewandten flüssigkeitsundurchlässigen Schicht und einem zwischen diesen Schichten befindlichen mehrschichtigen absorbierenden Kern besteht. Der Kern weist dabei eine erste Schicht aus einem hydrophilen synthetischen Mikrofaservlies auf, welches von einem Vliesstoff niedriger Dichte partiell umhüllt ist und aus einer sich daran anschließenden zweiten Schicht aus hydrophilem synthetischem Mikrofaservlies, welche in Kontakt mit einer weiteren Schicht aus hoch wasserspeicherndem Material steht und die erste Kernschicht an deren Unterseite zumindest am nicht umhüllten Bereich mit der zweiten Kernschicht fest verbunden ist. In DE 694 10 637 T2 wird ein Absorptionskörper in einem Absorptionsprodukt offenbart, bei dem der Absorptionskörper einen Flüssigkeitserfassungsabschnitt und einen dazu benachbarten Flüssigkeitslagerabschnitt aufweist, wobei der Flüssigkeitserfassungsabschnitt wenigstens einen Schacht aufweist, der an dem zu erwartenden Nässbereich des Absorptionskörpers angeordnet ist. Der Schacht erstreckt sich dabei in und durch den Flüssigkeitslagerabschnitt und steht in Flüssigkeitsverbindung mit einer Flüssigkeits-Dochtwirkungsschicht, die unter der Flüssigkeitslagerschicht angeordnet ist, wodurch die Flüssigkeit auch beim wiederholten Nässen durch eine Dochtwirkung in Richtung der unbenutzten Abschnitte des Absorptionskörpers verteilt werden kann.

Aus der EP 1 022 009 A2 ist eine dreilagige Unterlage für ein Hygieneprodukt bekannt, wobei zwischen einer ersten und einer zweiten Lage eine Mittellage hoher Dichte angeordnet ist. Die dreilagige Unterlage soll ein Austreten von Flüssigkeit aus dem Hygieneprodukt verhindern. Aus der U.S. 5,928,209 geht eine Unterlage hervor, die mehrlagig aus verschiedenen Vliesschichten aufgebaut ist. Die Unterlage bildet eine Sperrschicht für Flüssigkeit, die gleichzeitig wasserdampfdurchlässig bleiben soll. Aus der U.S. 5,643,239 ist es für eine Windel bekannt, einen Aufbau zu wählen, der eine Oberlage, einen Kern, eine Sperrlage sowie eine Unterlage aufweist. Die Sperrlage sowie die Unterlage sind wasserdampfdurchlässig sowie derartig ausgebildet, dass sie eine Sperre gegenüber Flüssigkeit bilden. Eine Sperrwirkung gegenüber Flüssigkeit soll gemäß dieses Standes der Technik dadurch erhöht werden, dass eine weitere hydrophobe Barrierelage aus Vliesstoff zwischen der Sperrlage und der hydrophoben Unterlage angeordnet wird.

Aufgabe der vorliegenden Erfindung ist es, ein flüssigkeitsaufnehmendes atmungaktives Wegwerfprodukt, beispielsweise einen Einwegartikel, insbesondere Hygieneprodukt, zur Verfügung zu stellen, welches in der Lage ist, unter Einsatzbedingungen einen Durchfluss an Flüssigkeit im wesentlichen zu vermeiden.

Diese Aufgabe wird gelöst mit einem flüssigkeitsaufnehmenden Wegwerfprodukt mit den Merkmalen des Anspruches 1.

Weitere vorteilhafte Ausgestaltungen und Weiterbildungen sind in den abhängigen Ansprüchen angegeben.

Ein flüssigkeitsaufnehmendes Wegwerfprodukt, insbesondere Hygieneprodukt, hat eine Oberlage und einen flüssigkeitsaufnehmenden und -speichernden Kern. Dem Kern nachgeordnet befindet sich eine Flüssigkeitsverteilungs- und Speicherungslage zur Aufnahme von Flüssigkeit, die aus dem flüssigkeitsaufnehmenden Kern bei Druckausübung auf diesen austritt.

Die Flüssigkeitsverteilungs- und Speicherungslage ist in der Lage, die aus dem Kern ausgetretene Flüssigkeit zu speichern. Dazu verteilt sich bei Druckausübung auf nur einen Teil des Kern und damit auch auf nur einen Teil der nachgeordneten Flüssigkeitsverteilungs- und Speicherungslage die Flüssigkeit vorzugsweise seitlich in der Flüssigkeitsverteilungs- und Speicherungslage anstatt nun auch aus der nachgeordneten Lage auszutreten. Im folgenden wird die Flüssigkeitsverteilungs- und Speicherungslage mit Flüssigkeitsverteilungslage bezeichnet.

Gemäß einer Ausgestaltung weist die Flüssigkeitsverteilungslage bei Druckausübung auf einen Bereich des Kerns einen geringeren Strömungswiderstand bezüglich einer Verteilung der Flüssigkeit in der Flüssigkeitsverteilungslage auf gegenüber einem Strömungswiderstand bezüglich eines Austretens der Flüssigkeit aus der Flüssigkeitsverteilungslage. Diese Ausgestaltung ermöglicht, dass die Gefahr einer Verschmutzung von beispielsweise Unterwäsche vermieden wird, wenn Druck beispielsweise im Sitzen auf den flüssigkeitsspeichernden Kern ausgeübt wird. Durch die Flüssigkeitsverteilungslage wird ermöglicht, dass auf den Kern wirkende Druckspitzen und dadurch verursachte aus dem Kern austretende Flüssigkeitsströme aufgefangen und gespeichert werden. Vorzugsweise wird die Flüssigkeit nur zwischengespeichert und strömt nach Druckentlastung in den Kern zurück. Gemäß einer Weiterbildung weist die Flüssigkeitsverteilungslage ein Volumen auf, das in der Lage ist, eine Zwischenspeicherung allein in diesem Volumen zu gewährleisten. Einzelne Fasern der Flüssigkeitsverteilungslage sind vorzugsweise nicht in der Lage, eine Flüssigkeit aufzunehmen. Insbesondere sind die Fasern hydrophob.

Eine Weiterbildung sieht vor, dass eine an die Flüssigkeitsverteilungslage angrenzende Lage einen höheren hydrostatischen Druckwiderstand aufweist als die Flüssigkeitsverteilungslage. Beispielsweise kann durch ein oder mehrere Sperrlagen gegenüber der Flüssigkeit der Strömungswiderstand erhöht werden kann. Die Sperrlage kann zwischen dem Kern und der Flüssigkeitsverteilungslage, der Flüssigkeitsverteilungslage nachgeordnet oder auch beides sein. Die angrenzende Lage kann eine Sperrlage sein, wie sie im oben geschilderten Stand der Technik eingesetzt wird oder auch ein wasserdampfdurchlässiger Film. Vorzugsweise hat die Flüssigkeitsverteilungslage einen größeren mittleren Porendurchmesser als eine zur Flüssigkeitsverteilungslage angrenzende Lage. Zum einen ist die Flüssigkeitsverteilungslage dadurch mit einem größeren Stauvolumen ausrüstbar. Zum anderen kann über eine Einstellung der Anzahl und der Größe der Poren eine gezielte Druckminderung im flüssigkeitsaufnehmenden Wegwerfprodukt bei aufgebrachtem Druck und fließender Flüssigkeit erzielt werden.

Gemäß einer weiteren Ausgestaltung ist der Aufbau der Flüssigkeitsverteilungslage derart, dass die vom Kern abgegebene Flüssigkeit innerhalb der Flüssigkeitsverteilungslage verteilt wird. Der auf den Kern ausgeübte Druck wird gleichzeitig auch auf die Flüssigkeitsverteilungs- und Speicherungslage ausgeübt. Bei einem Zusammenpressen des Kerns und dabei Auslaufen der Flüssigkeit bietet die Flüssigkeitsverteilungslage im Austrittsbereich der Flüssigkeit einen geringeren Widerstand gegenüber einer Verteilung in dieser Lage selbst. Gemäß einer Weiterbildung ist die Flüssigkeitsverteilungslage so ausgebildet, dass vermieden wird, dass Flüssigkeit bis zu einer eventuell der Flüssigkeitslage nachgeordneten Unterlage gelangt. Gemäß einer weiteren Ausgestaltung gelangt zwar die Flüssigkeit zwar bis an die Unterlage. Dort jedoch wird die Flüssigkeit seitlich in der Flüssigkeitsverteilungslage verteilt, anstatt durch die Unterlage hindurchzutreten. Die Unterlage bildet eine Außenlage des Wegwerfprodukts.

Die Flüssigkeitsverteilungslage hat den weiteren Vorteil, dass durch diese zumindest eine Dampfdurchlässigkeit bei gleichzeitigem Schutz gegenüber einem Durchnässen vorhanden ist. Vorzugsweise ist die Flüssigkeitsverteilungslage auch luftdurchlässig. Ebenso kann eine optional nachgeordnete Unterlage wasserdampfdurchlässig und/oder luftdurchlässig sein. Beispielsweise kann ein poröser Film verwendet werden.

Gemäß einer weiteren Ausgestaltung weist die Flüssigkeitsverteilungslage einen uneinheitlichen Aufbau auf. Dieser uneinheitliche Aufbau ist beispielsweise erzielbar, indem die Flüssigkeitsverteilungslage eine erste mittlere Porengröße und eine zweite mittlere Porengröße aufweist, wobei die erste Porengröße von der zweiten Porengröße verschieden ist. Auch besteht die Möglichkeit, dass die Flüssigkeitsverteilungslage Bereiche mit unterschiedlichen Dichten und/oder unterschiedlich großen mittleren Zwischenräumen hat. Diese Bereiche können fließend ineinander übergehen oder aber auch über beispielsweise Grenzflächen voneinander getrennt sein. Die Flüssigkeitsverteilungslage kann weiterhin Bereiche mit unterschiedlichen Faser- und/oder Filamentdurchmessern aufweisen. Gemäß einer Weiterbildung weist die Flüssigkeitsverteilungslage zwei und mehr Schichten auf, die beispielsweise fest miteinander verbunden sind. Weiterhin kann das Benetzungsverhalten der Flüssigkeitsverteilungslage eingestellt werden, zum Beispiel über das verwendete Material, über eine entsprechende Beschichtung oder Benetzung oder anderes. Die Benetzungsfähigkeit kann auch über einen Querschnitt der Flüssigkeitsverteilungslage unterschiedlich sein, beispielsweise herabgesetzt werden in Richtung einer Außenseite des Wegwerfproduktes. Gemäß einer Ausgestaltung der Flüssigkeitsverteilungslage weist diese einen Gradienten bezüglich des zur Verfügung gestellten Volumens über einen Querschnitt durch die Flüssigkeitsverteilungslage auf. Materialien für die Flüssigkeitsverteilungslage sind Vliese.

Vorzugsweise ist die Flüssigkeitsverteilungslage in verschiedenen Bereichen so unterschiedlich gestaltet, dass ein aufgebrachter Druck in der Flüssigkeitsverteilungslage ungleichmäßig verteilt wird. Dieses ermöglicht eine gezielte Verteilung der Flüssigkeit: wird der Widerstand in Richtung auf die Unterlage erhöht, weicht die Flüssigkeit seitlich aus. Die Flüssigkeitsverteilungslage weist dafür ein ausreichendes Speichervolumen auf. Gemäß einer Weiterbildung hat die Flüssigkeitsverteilungslage in einem ersten Gebiet bevorzugter Druckbeaufschlagung des flüssigkeitsaufnehmenden Wegwerfproduktes unter Nutzung einen höheren Strömungswiderstand gegenüber einem zweiten, benachbarten Gebiet der Flüssigkeitsverteilungslage.

Gemäß einer Weiterbildung weist die Flüssigkeitsverteilungslage eine gewisse Steifigkeit auf. Dazu kann das verwendete Material der Flüssigkeitsverteilungslage eine Hauptrichtung an Steifigkeit aufweisen. Vorzugsweise verläuft diese in etwa entlang der Dicke der Flüssigkeitsverteilungslage. Eine weitere Ausgestaltung sieht vor, dass der Kern eine geringere Steifigkeit hat als die Flüssigkeitsverteilungslage. Vorzugsweise hat die Flüssigkeitsverteilungslage eine hochporöse Struktur, die einer in der Flüssigkeitsverteilungslage befindlichen Flüssigkeit eine freiere Bewegung erlaubt gegenüber einer Flüssigkeit, die sich im Kern befindet. Der Kern weist beispielsweise zusätzlich Superadsorbent, Zellulosematerial oder sonstiges auf. Eine Weiterbildung sieht vor, dass der Kern Kapillarkräfte auf eine Flüssigkeit wirken lässt, während die Flüssigkeitsverteilungslage vorzugsweise über keine oder nur äußerst geringe Kapillarkräfte verfügt.

Das Wegwerfprodukt, beispielsweise eine Windel, eine Damenbinde oder ähnliches, ist an die Nutzung körperergonomisch angepasst. Während des Tragens durchlebt das Wegwerfprodukt dabei verschiedene Zustände. Einer dieser Zustände ist die Ausübung vermehrten Druckes, beispielsweise im Sitzen oder Anlehnen der betreffenden. Körperstelle in Verbindung mit dem Wegwerfprodukt. Aufgrund der Körperergonomie ist vorhersagbar, dass beispielsweise bei Windeln bestimmte Bereiche der Gesäßpartie besonders druckbeaufschlagt sind. Durch eine entsprechende Anpassung von Widerständen der Flüssigkeitsverteilungslage an diese Sitzweise des Wegwerfproduktes ist eine entsprechende von aus dem Kern ausströmender Flüssigkeit in die Flüssigkeitsverteilungslage ermöglicht.

Gemäß einer weiteren Ausbildung weist die Flüssigkeitsverteilungslage einen Kapillareffekt auf. Dieser Kapillareffekt bildet sich vorzugsweise erst beabstandet vom Kern in der Flüssigkeitsverteilungslage aus. Beispielsweise ist der Kapillareffekt zumindest teilweise in Richtung der Unterlage ausgerichtet. Von dort kann gemäß einer Weiterbildung der Kapillareffekt so wirken, dass die Flüssigkeit seitlich verteilt wird. Durch eine entsprechende Ausgestaltung der Flüssigkeitsverteilungslage wird der Kapillareffekt vorzugsweise so erzielt, dass die an den Kern direkt oder mittelbar angrenzende Oberfläche der Flüssigkeitsverteilungslage austretende Flüssigkeit diese zuerst nur aufnimmt. Über eine entsprechende kanalähnliche Gestaltung und Verteilung innerhalb der Flüssigkeitsverteilungslage wird erst in einigem Abstand zum Kern der Kapillareffekt wirksam. Der Kapillareffekt kann durch entsprechende Formgestaltung der Fasern oder Filamente oder sonstiger Materialien der Flüssigkeitsverteilungslage erzielt werden. Vorzugsweise hat die Flüssigkeitsverteilungslage in einem seitlichen Bereich eine Kapillarwirkung, die Flüssigkeit aus der Richtung einer potentiell vorhanden Unterlage zurück zu dem Kern führt. Der Kern selbst hat gemäß einer Weiterbildung ebenfalls eine Kapillarwirkung. Diese ist vorzugsweise in unmittelbarer Nähe zur Flüssigkeitsverteilungslage wirksam. Flüssigkeit kann so in der Flüssigkeitsverteilungslage zwischengespeichert werden und anschließend, bei entsprechender Verteilung, wieder in den Kern zurückfließen.

Gemäß einer Weiterbildung hat die Flüssigkeitsverteilungslage eine Wirkungsweise auf auftreffende Flüssigkeit, die bezogen auf einen Querschnitt mit einem ersten hydrophob wirkenden Bereich, einem zweiten hydrophil wirkenden Bereich und einem dritten hydrophob wirkenden Bereich beschrieben werden kann.

Um ein seitliches Austreten von Flüssigkeit aus dem Kern zu vermeiden, wird die Flüssigkeitsverteilungslage vorteilhafterweise so angeordnet, dass sie seitlich über den Kern hinausragt. Dazu kann die Flüssigkeitsverteilungslage den Kern zumindest teilweise oder auch ganz umschließen.

Gemäß einem weiteren Gedanken, der auch eigenständig bezüglich eines anderen Vliesstoffes, beispielsweise eines Verbundes aus Spinnvlies, schmelzgeblasenem Vlies und/oder Spinnvlies (SMS-Material), weiterverfolgt werden kann, weist die Flüssigkeitsverteilungslage ein durchflusshemmendes Mittel auf. In Kontakt mit einer Flüssigkeit wird das Mittel vorzugsweise aktiviert. Eine Aktivierung ist beispielsweise durch Agglomerationskörper möglich, an der die Flüssigkeit gebunden oder durch die eine Verfestigung innerhalb der Flüssigkeit ausgelöst wird. Ein derartiger Vorgang ist auch durch eine entsprechende Keimwirkung erzielbar, die in die Flüssigkeit bei Einströmen und Durchströmen der Flüssigkeitsverteilungslage eingebracht werden kann. Je nach Einsatzgebiet des Wegwerfproduktes können verschiedene Substanzen eingesetzt werden. Wird das Wegwerfprodukt in Form eines Hygieneproduktes verwendet, das in Kontakt mit einer Körperflüssigkeit, insbesondere Blut, tritt, sind auf Eiweißkörper, Hämoglobin oder andere Bestandteile wirkende, insbesondere aktivierbare Substanzen einsetzbar. Diese können beispielsweise ein Verklumpen des Blutes ermöglichen.

Des weiteren besteht die Möglichkeit, dass das durchflusshemmende Mittel eine Saugfunktion besitzt. Aufgrund dieser wird die Flüssigkeit am Mittel gebunden. Das Mittel selbst kann quellfähig sein, wie beispielsweise es bei superabsorbierenden Materialien bekannt ist. Ebenfalls einsetzbar ist geruchskontrollierendes Material oder andere, eine bestimmte Wirkung aufweisende Materialien, zum Beispiel Zeolithe, Silikate, pH-Regulatoren und/oder Kohlenstoff.

Vorzugsweise ist die Flüssigkeitsverteilungslage mit einem flüssigkeitsaufnehmendem Volumen ausgestattet, dass beispielsweise zwischen 1/10 und 1/3 von demjenigen des Kerns beträgt. Ein größeres Volumen wie auch ein kleineres Volumen kann vorgesehen werden. Dieses ist beispielsweise abhängig von der Anwendung des Wegwerfproduktes, des Aufbaus und der unterschiedlichen Eigenschaften der einzelnen verwendeten Lagen. Vorzugsweise erfolgt die eigentliche Flüssigkeitsaufnahme und Flüssigkeitsspeicherung vom Kern. Nur unter besonderen Umständen, wie beispielsweise Druck auf den Kern und dadurch austretende Flüssigkeit, tritt die Flüssigkeitsverteilungslage in ihre Funktion als Speicherung. Durch ein entsprechend vorgesehenes Volumen kann weiterhin gleichzeitig sichergestellt werden, dass die Flüssigkeitsverteilungslage mit ihrer Speicherfunktion auch ein Notreservevolumen zur Verfügung stellt. Dieses kann beispielsweise nötig sein, wenn der Kern vollständig gesättigt ist. Die überschüssige Flüssigkeit wird durch die Flüssigkeitsverteilungslage entsprechend gespeichert, ohne dass sie an die eventuell vorgesehene Unterlage weitergeführt wird. Ein derartiger Zustand tritt beispielsweise bei einer längeren Tragezeit des Wegwerfproduktes als Hygieneprodukt auf, bei einer Damenbinde, oder bei einem zugeführten Flüssigkeitsvolumen, das oberhalb gewisser Grenzwerte liegt. Damit steht im Zusammenhang ein Zustrom an Flüssigkeit während eines kurzen Zeitraumes. Vorzugsweise wird die Flüssigkeitsverteilungslage bei Windeln für Neugeborene, also für die ersten Wochen nach der Geburt verwendet. Der Aufbau der einzelnen Lagen ist vorzugsweise derart, dass nach Druckentlastung zumindest ein Teil der Flüssigkeit in der Flüssigkeitsverteilungslage wieder in den Kern zurückströmt. Beispielsweise kann dazu das Betreben nach einer Gleichgewichtsverteilung der Flüssigkeit ausgenutzt werden.

Unterstützt wird die Funktion der Flüssigkeitsverteilungslage, indem zwischen dieser und dem Kern eine Sperrlage angeordnet ist, die im folgenden mit Sperrschicht bezeichnet wird. Diese Sperrschicht bildet einen Widerstand zwischen dem Kern und der Flüssigkeitsverteilungslage. Gemäß einer Ausgestaltung gelingt es der Flüssigkeit erst bei Überschreiten eines von außen aufgebrachten Druckes, durch die Sperrschicht hindurch in die Flüssigkeitsverteilungslage zu gelangen. Vorzugsweise hat die Sperrschicht einen Strömungswiderstand, der gegenüber einer Flüssigkeit größer ist als derjenige Strömungswiderstand, den die Flüssigkeitsverteilungslage gegenüber dieser Flüssigkeit selbst aufweist. Die flüssigkeitsdurchlässige Sperrschicht ist beispielsweise ein Vliesstoff wie zum Beispiel ein SMS-Material, eine SM-Lage, ein kardiertes Material, eine Spinnvlieslage oder andere im Stand der Technik bekannte Barrierematerialien wie beispielsweise ein Film, ein mikroporöser Film oder ein Verbund aus Film und Vliesstoff oder ähnliches.

Gemäß einer weiteren Ausgestaltung, die auch mit anderen kombinierbar ist, weist das Wegwerfprodukt als Sperrschicht einen wasserundurchlässigen Film auf, an den eine Flüssigkeitsverteilungslage angrenzt. Die unter Druck stehende Flüssigkeit kann den Film nicht durchdringen und tritt seitlich aus dem Kern aus. Zumindest in diesem Bereich ist mindestens eine Flüssigkeitsverteilungslage angeordnet, beispielsweise in Form eines Streifens oder als zumindest teilweise Umhüllung von mindestens einem seitlichen Ende des Kerns. Die Flüssigkeitsverteilungslage oder -lagen speichern vorzugsweise die Flüssigkeit zwischen. Nach Druckentlastung des Kerns kann die Flüssigkeit wieder aus der Flüssigkeitsverteilungslage in den Kern zurückfließen.

Eine Weiterbildung sieht vor, dass eine Unterlage des Wegwerfproduktes und/oder eine Sperrschicht zumindest teilweise hydrophob ist.

Gemäß einem weiteren Gedanken betrifft die Erfindung auch eine Lage für ein flüssigkeitsaufnehmendes Wegwerfprodukt. Die Lage ist eine Flüssigkeitsverteilungsund Speicherungslage, wie sie oben beschrieben ist. Vorzugsweise hat diese Lage einen Strömungswiderstand, der in einem Dickenabschnitt der Lage größer ist als in einem zum Dickenabschnitt direkt benachbarten Längsabschnitt der Lage, um eine Flüssigkeit in der Flüssigkeitsverteilungslage zu verteilen. Ein derartiger Strömungswiderstand vermeidet, dass die in die Lage eintretende Flüssigkeit vorzugsweise bestrebt ist, durch diese Lage hindurch zu gelangen. Der Strömungswiderstand wird beispielsweise mit einer ungleichmäßigen Ausgestaltung der Flüssigkeitsverteilungs- und Speicherungslage erzielt. Mittel dazu sind beispielsweise oben in der Beschreibung angegeben.

Eine weitere Möglichkeit einer gezielten Flüssigkeitssteuerung besteht darin, dass die Lage ein Verbindungsmuster aufweist, welches den Strömungswiderstand beeinflußt, vorzugsweise einstellt. Das Verbindungs- oder auch Bondingmuster kann einen Bereich eingrenzen, der ohne Verbindungsstellen ausgestaltet ist. Dieser Bereich hat vorzugsweise einen geringeren Strömungswiderstand und vorzugsweise auch einen geringeren Verteilungswiderstand. Beispielsweise weist ein zu einem Verbindungsbereich mit einem entsprechenden Bondingmuster angrenzender Bereich aufgrund der Verbindungsstellen engere Poren beziehungsweise engere Zwischenräume auf. Diese erhöhen den Strömungswiderstand, da auch die Verbindungsstellen selbst den Anteil an durchlassfähiger Fläche vermindern. Das Bondingmuster selbst kann punktmäßig, linienförmig, fischgrätmusterähnlich, großflächig, kleinflächig, Mischungen von diesen oder ähnliches sein. Es kann mittels entsprechend ausgeformter Kalanderwalzen, mittels Ultraschallverfahren, mittels Klebemittel, mittels des Materials der Fasern oder Filamente selbst oder anderen Werkzeugen oder Materialien gebildet werden. Die Verbindung kann auch so eingestellt werden, dass dadurch ein Kapillareffekt erzielt wird. Vorzugsweise wird durch ein Bondingmuster der Strömungswiderstand hin zu den Seiten der Lage eingestellt.

Gemäß einer Ausgestaltung der Lage als Flüssigkeitsverteilungs- und Speicherungslage für ein Hygieneprodukt hat die Lage eine Dicke von mindestens 0,05 mm, insbesondere von mindestens 0,1 mm und vorzugsweise von 0,5 mm. Sie kann auch über 1 mm dick sein. Die Dicke kann in Abhängigkeit des Ortes auch variieren.

Des weiteren stellt die Erfindung ein Verfahren zum Testen einer Flüssigkeitsverteilungs- und Speicherungslage für ein flüssigkeitsaufnehmendes Wegwerfprodukt wie oben beschrieben bzw. für eine oben beschriebene Lage zur Verfügung. Das Verfahren weist die folgenden Schritte auf:
- Auftragen einer Flüssigkeit auf ein mehrlagigen Gebildes, das die Flüssigkeitsverteilungslage enthält, wobei die Flüssigkeitsverteilungslage an eine Testoberfläche angrenzt, die flüssigkeitsaufnehmend ist,
- Aufbringen eines definierten Drucks von mindestens 2000 N/m² für einen Zeitraum von mindestens 5 min auf das mehrlagige Gebilde und
- Ermitteln von zumindest des Flüssigkeitsdurchgangs in die Flüssigkeitsverteilungslage.

In einem weiteren Schritt wird nicht nur der Flüssigkeitsdurchgang gemessen und gegebenenfalls bewertet. Vielmehr wird auch der Verschmutzungsgrad ausgehend von dem Flüssigkeitsdurchgang bewertet, der durch die Flüssigkeitslage hindurchgeht. Dieses erfolgt vorzugsweise dadurch, dass an das mehrlagige Gebilde die Testoberfläche angrenzt. Aufgrund der definierten Testbedingungen ist feststellbar, ob die Testoberfläche eine Verschmutzung aufweist. Diese ergibt sich aufgrund der aus dem Kern austretenden Flüssigkeit bzw. aus der Flüssigkeitsverteilungslage austretenden Flüssigkeit. Dadurch kann geprüft werden, ob ein Hygieneprodukt in Kontakt mit Körperwäsche letztere nicht verschmutzen wird.

Der Test kann variiert werden, indem die Zeit wie auch der Druck verändert werden. Beispielsweise kann der Druck etwa 22 kN/m² betragen und der Zeitraum etwa 10 Minuten. Vorzugsweise wächst der Druck an bei einer Verkürzung des Zeitraums der Druckbeaufschlagung.

Des weiteren kann die Flüssigkeitsverteilungs- und Speicherungslage die folgenden Eigenschaften einzeln oder in Kombination aufweisen, insbesondere auch in Kombination mit Eigenschaften des Kerns:
Geruchskontrolle, Fluidadhäsion, Fluiddehäsion, Alkoholabweisung, Hydrophilie, Hydrophobie, Verdünnung einer Flüssigkeit, Verdickung einer Flüssigkeit, Erstarrung einer Flüssigkeit, Koagulation einer Flüssigkeit, Niederschlag oder Austrocknen der Flüssigkeit.

Insbesondere kann die Flüssigkeitsverteilungslage bewegungsunfähig machende Mittel, vorzugsweise immobilisierende Mittel, wie beispielsweise oben beschrieben, an bestimmten Regionen beispielsweise im Randbereich aufweisen. Dadurch wird ein Austreten von Flüssigkeit aus der Flüssigkeitsverteilungslage vermieden. Auch besteht die Möglichkeit, eine entsprechende Eigenschaft in der Flüssigkeitsverteilungslage in einem Randbereich vorzusehen, der zur Unterlage hin ausgerichtet ist. Dieser hat die Funktion, als Sperre zu wirken, beispielsweise durch ein Verschließen von ansonsten für zumindest Wasserdampf durchlässige Öffnungen. Des weiteren kann ein derartiges Mittel in der Flüssigkeitsverteilungslage so angeordnet sein, dass durch die dadurch bewegungsunfähige Flüssigkeit eine gezielte Strömungswiderstandserhöhung erzeugt wird. Beispielsweise ist damit ein mit Flüssigkeit gesättigter Bereich sperrbar. Zusätzlich nachströmende Flüssigkeit wird daraufhin in andere Bereiche der Flüssigkeitsverteilungslage umgeleitet bzw. umgelenkt. Beispielsweise kann die Flüssigkeitsverteilungslage zumindest teilweise entsprechend behandelt sein, beispielsweise mittels einer oleophoben Behandlung, einer hydrophoben Behandlung und/oder oberflächenspannungsabsenkenden Behandlung, beispielsweise einer HO+ Behandlung. Die Wirkungen der Behandlung können insbesondere physikalisch/chemisch intramolekular sein.

Vorzugsweise wird das auch als funktionsgebende Substanz bezeichenbare Material entsprechend eines vorgebbaren Musters an voneinander beabstandeten Punkten oder Orten in und/oder auf der Flüssigkeitsverteilungslage eingebracht bzw. aufgetragen. Diese Punkte oder Stellen sind so ausgewählt, dass beispielsweise eine Eigenschaft wie eine Luftdurchlässigkeit der Flüssigkeitsverteilungslage im Falle einer Aktivierung der Substanz möglichst gering gestört wird. Beispielsweise weist die Flüssigkeitsverteilungslage soviel zusätzliche Substanz auf, dass bei einer Sättigung der Flüssigkeitsverteilungslage eine Luftdurchlässigkeit um maximal 70%, insbesondere bis 50 % gegenüber einer Flüssigkeitsverteilungslage ohne gespeicherte Flüssigkeit abnimmt. Die Luftdurchlässigkeit wird vorzugsweise mittels der Testmethode ERT 140.1-81 gemessen. Die Luftdurchlässigkeit der Flüssigkeitsverteilungslage ist vorzugsweise größer gegenüber derjenigen der Unterlage. Die Luftdurchlässigkeit der Unterlage beträgt gemäß eines Ausführungsbeispiels bis zu 3000 l/m²s. Ist zwischen dem Kern und der Flüssigkeitsverteilungslage eine zusätzliche Sperrschicht angeordnet, so hat die Sperrschicht vorzugsweise eine Luftdurchlässigkeit, die bis zu 1000 I/m²s beträgt. Gemäß einem weiteren Ausführungsbeispiel weist die Unterlage einen Wassersäulenwert gemäß DIN 53886 von mindestens 8 cm, die Sperrschicht einen Wert von mindestens 7 cm auf. Der Wassersäulenwert der Flüssigkeitsverteilungslage ist vorzugsweise geringer als der geringste der beiden Werte, während der Wassersäulenwert der Sperrschicht vorzugsweise größer als derjenige der Unterlage ist.

Eine oben schon erwähnte mittlere Porengrößenverteilung sowie das zur Verfügung stehende Speichervolumen der Flüssigkeitsverteilungs- und Speicherungslage werden vorzugsweise über ein Coulter Porometer 2 ermittelt. Gemäß einer Ausgestaltung sind die durchschnittliche Porengröße sowie das Gesamtspeichervolumen vorzugsweise größer als bei der Unterlage wie auch der Sperrschicht.

Eine Weiterbildung sieht vor, dass die Flüssigkeitsverteilungs- und Speicherungslage einen Druckwiderstand aufweist, der verschieden ist gegenüber derjenigen der Sperrschicht und/oder der Unterlage. Der Druckwiderstand gibt das Verhalten der jeweiligen Lage auf mechanisch aufgebrachten Druck von außen an. Der Druckwiderstand wird beispielsweise mittels eines Schiefer Kompressometers gemessen. Gemäß einer Ausgestaltung ist der Druckwiderstand der Flüssigkeitsverteilungslage geringer als derjenige der Sperrschicht und der Unterlage. Gemäß einer anderen Ausgestaltung ist der Druckwiderstand der Flüssigkeitsverteilungslage größer als zumindest derjenige der Sperrschicht oder zumindest der Unterlage. Insbesondere kann die Flüssigkeitsverteilungslage auch einen anderen Druckwiderstand aufweisen als der Kern. Vorzugsweise ist der Druckwiderstand des Kerns geringer als derjenige der Flüssigkeitslage.

Wird beispielsweise eine Flüssigkeitsverteilungs- und Speicherungslage zwischen einer Sperrlage und einer Unterlage angeordnet, die beide als Flüssigkeitssperren dienen, weist die dazwischen angeordnete Flüssigkeitsverteilungslage im Mittel größere Poren auf als zumindest eine der beiden anderen Lagen. Vorzugsweise sind die Poren sogar größer als diejenigen von beiden Lagen. Bei Druckausübung auf eine der beiden Lagen wandert die Flüssigkeit in die Flüssigkeitsverteilungslage, anstatt durch die Unterlage hindurchgedrückt zu werden. Vorzugsweise wird die Flüssigkeitsverteilungslage zumindest teilweise im Inneren oder auf der Oberfläche hydrophil ausgestattet, um die Verteilung zu unterstützen.

Eine Weiterbildung sieht vor, dass die Sperrlage, die den Kem von der Flüssigkeitsverteilungslage trennt, zumindest im wesentlichen flüssigkeitsundurchlässig ist, beispielsweise mittels eines mikroporösen Films, der durchlässig für Wasserdampf ist, mittels eines luftdurchlässigen Films oder mittels eines unporösen Films. Eine andere Weiterbildung sieht vor, die Sperrlage flüssigkeitsdurchlässig zu gestalten, zum Beispiel ein hydrophiles Vlies, ein Spinnvlies, ein kardiertes oder auch ein SMS-Vlies zu verwenden. Ebenfalls kann ein luftdurchlässiger Film eingesetzt werden, der perforiert ist. Die Perforation ist beispielsweise so durchgeführt, dass kegelähnliche Gebilde mit einer Öffnung an der Spitze entstehen. Bei Druck auf die Spitze kann die Öffnung verschlossen werden. Der Kegel ist vorzugsweise in Richtung Kem angeordnet.

Eingedrungene Flüssigkeit weicht in der Flüssigkeitsverteilungslage bei partiellem Druck seitlich in weniger stark druckbeanspruchte Bereiche aus. Vorzugsweise ist ein Widerstand in der Flüssigkeitsverteilungslage nicht höher als der Widerstand, der der Flüssigkeit beim Durchschreiten der größten Pore einer Sperrlage, insbesondere der Unterlage, entgegengesetzt wird. Gemäß einer Weiterbildung bleibt die Flüssigkeitsverteilungslage bei aufgenommener Flüssigkeit und/oder unter Druck luftdurchlässig. Eine andere Ausgestaltung sieht vor, dass die Flüssigkeitsverteilungslage einen Film aufweist, der mikroporös und/oder perforiert ist. Der Film hat vorzugsweise zumindest an einer Oberfläche Härchen, die insbesondere auch hohl sein können. Die Härchen haben beispielsweise eine Länge, die mindestens 250 µm beträgt, vorzugsweise etwa 400 µm. Gemäße einer ersten Ausgestaltung sind die Härchen an einer Oberfläche des Films angeordnet, die vom Kern wegweist. Gemäß einer zweiten Ausgestaltung sind Härchen an beiden Oberflächen de Films angeordnet.

Beispielsweise im Falle eines Damenbindenproduktes hat die Flüssigkeitsverteilungslage vorzugsweise einen Durchnässungswert, der kleiner oder gleich 0.5 g, insbesondere bevorzugt geringer als 0.3 g ist. Der Durchnässungswert wird gemäß Testmethode Corovin "Wet Through" ermittelt.

Insbesondere wird eine Verschmutzung bzw. Fleckenbildung dadurch unterbunden, dass die Flüssigkeitsverteilungslage mit einer Sperrschicht und/oder mit einer Unterlage verbunden wird, die einen Wassersäulenwert von in etwa 15 cm oder mehr entsprechend DIN 53886 aufweist, wobei der Wert auch geringer ausfallen kann. Die Flüssigkeitsverteilungslage weist ein Vliesmaterial auf, welches Spinnvlies, wasserstrahlverfestigten Vliesstoff, schmelzgeblasenen Vliesstoff, Krempelvliesstoff, hochvoluminöses Vlies, beispielsweise hochvoluminöses Vlies aus Krempelvliesstoff, Schaum, vorzugsweise offenporigen Schaum, beispielsweise Schaumstoff, Cellulosefasern, Mischungen aus diesen Materialen in Form von Durchmischungen oder unterschiedlichen Lagen, einzeln oder im Verbund aufweist.

Die Flüssigkeitsverteilungslage weist gemäß einer Weiterbildung ein oder mehrere Lagen bzw. Materialien bzw. Gemenge auf. Werden Fasermaterialien verwendet, ist es bevorzugt, dass der mittlere Durchmesser der Fasern der Flüssigkeitsverteilungslage größer ist als derjenige von Fasern der Unterlage und/oder der Sperrschicht. Bevorzugt beträgt der Faserdurchmesser einen Wert von 9 µm und mehr, insbesondere ist er größer als 15 µm.

Das Flächengewicht der Flüssigkeitsverteilungslage, gemessen gemäß ERT 40.3-90, liegt vorzugsweise in einem Bereich zwischen 5 g/m² und 200 g/m² und mehr, bevorzugt zwischen 8 g/m² bis 50 g/m², insbesondere in einem Bereich zwischen 10 g/m² bis 31 g/m². Bei einer Damenbinde beträgt ein bevorzugtes Flächengewicht etwa 10 g/m² bis 60 g/m². Die Flüssigkeitsverteilungslage weist vorzugsweise zumindest ein thermoplastisches Polymer oder Copolymer auf, welches beispielsweise enthalten ist in der Gruppe aus Polyolefinen, Polyamiden, Polyester, Polyether, Polyesterether, wobei Polyolefine Homopolymere und Copolymere auf der Basis von Ethylen und/oder Propylen mitumfassen.

Aus der folgenden Tabelle sind Materialien zu entnehmen, die sich für einen Einsatz im Wegwerfprodukt, insbesondere bei einer Damenbinde, als geeignet herausgestellt haben. Die Tabelle 1 gibt nur Beispiele wieder, ohne weitere Materialien. auszuschließen oder aber die angegebenen Parameter als Grenzen zu verstehen. Vielmehr sind die Gewichtsangaben nach oben wie auch nach unten variabel gestaltbar.

**Tabelle 1:**

| **Lage** | **Typ** | **Flächengewicht (g/m**^{**2**}**)*,* Angabe beispielhaft** |
|---|---|---|
| CB1 | SMMS | 16 |
| CB2 | SM | 25 |
| CB3 | M | 16 |
| CB4 | SMS | 18 |
| CB5 | SMS | 24 |
| CB6 | SMS | 11 |
| SP1 | CH | 50 |
| SP2 | S (2.5 dtex) | 20 |
| SP3 | S ( 4 dtex) | 20 |
| BS1 | SM | 27.5, ein Flourcarbonadditiv enthaltend |
| BS2 | SM | 27.5 |
| BS3 | SM | 27.5. ein metallorganisches Additiv enthaltend |
| BS4 | SF | 33 |
| BS5 | SMSF | 44 |
| BS6 | SM | 25 |

Abkürzungen: CB Sperrschicht (Core barrier); SP Flüssigkeits- und Verteilungslage (Spacer layer); BS Unterlage (Backsheet); S Spinnvlies (Spunbond); M Schmelzgeblasenes Vlies (Meltblown); CH kardiertes, heißluftverfestigtes Vlies (Carded Highloft); F Film

Es wird bei SM, SMS- beziehungsweise SMMS-Materialien für die Sperrschicht zwischen dem Kern und der Flüssigkeitsverteilungslage angestrebt, ein Spinnvliesgewicht je Lage zwischen 3 und 25 g/m² einzustellen. Bei mehreren Lagen können in etwa gleiche Flächengewichte vorhanden sein. Eine Weiterbildung sieht vor, dass ungleiche Flächengewichte vorhanden sind, beispielsweise ein SMS-Material, bei dem eine erste Spinnvlieslage ein um 50% bis 150% größeres Flächengewicht hat als eine zweite Spinnvlieslage. Der schmelzgeblasene Vliesanteil beträgt vorzugsweise zwischen 1 g/m² und 8 g/m².

Für die Unterlage wird beispielsweise ein Spinnvliesgewicht verwendet, welches vorzugsweise ein Flächengewicht aufweist, das zwischen 12 g/m² und 24 g/m² liegt. Ein verwendeter Film ist insbesondere mikroporös. Das Filmgewicht beträgt vorzugsweise 15 g/m² bis etwa 32 g/m². Gemäß einer Weiterbildung ist das Flächengewicht des Films größer als das Flächengewicht des ebenfalls verwendeten Vlieses, vorzugsweise um zwischen 12,5% und 100%.

Vorteilhafte Wegwerfprodukte wie Damenbinden ergeben sich insbesondere bei den folgenden Kombinationen von Materialien:

**Tabelle 2:**

| | |
|---|---|
| Unterlage oder Sperrschicht | BS1, BS2, BS4 |
| Flüssigkeitsverteilungs- und Speicherungslage | SP2, SP3 |
| Doppelte Flüssigkeitsverteilungs- und Speicherungslage mit verringerter Durchnässung | SP2 + SP3 |
| Sperrschicht und Flüssigkeitsverteilungs- und Speicherungslage mit verminderter Durchnässung | CB2 + SP2, CB2 + SP3 |
| Flüssigkeitsverteilungs- und Speicherungslage und Unterlage mit verminderter Durchnässung | SP3 + BS6 |

Tabelle 3 gibt beispielhaft einige Parameter an, die für ein Wegwerfprodukt, insbesondere eine Damenbinde, welches eine Flüssigkeitsverteilungs- und Speicherungslage hat, anwendbar sind.

**Tabelle 3:**

| Eigenschaft | Methode | | Bereich | |
|---|---|---|---|---|
| | | CB | SP | BS |
| Luftdurchlässigkeit | ERT 140.1-81 | CB < SP | > CB; BS | BS < SP |
| Flächengewicht (Basisgewicht) | ERT 40.3-90/DIN 53854 | 10-100 gsm vorzugsweise 11-50 gsm | 8-300 gsm vorzugsweise 10-70 gsm | 11-50 gsm, vorzugsweise 11-40 gsm |
| Faserfeinheit [µm], einzeln oder Gemisch | CM-101-A | < SP > 0,5µm, vorzugsweise 1-35 µm | > CB; BS > 9µm, vorzugsweise > 15 µm, vorzugsweise zwischen 15 und 38 µm | vorzugsweise zwischen 1 und 38 µm, insbesondere zwischen 1 und 10 µm und 15 und 38 µm |
| Wassersäule | DIN 53886 | CB > SP, vorzugsweise ≧ 7 cm, insbesondere >15cm | SP < CB; BS | BS > SP, vorzugsweise ≧ 7 cm, insbesondere >14cm |
| Porengröße | CM-126-17 | CB < SP | SP > CB; BS | BS < SP |
| Durchnässung | "Wet Through" Test Methode | vorzugsweise < 1g, insbesondere ≦ 0,5 g | Zum Beispiel mit CB oder BS: vorzugsweise < 1,0 g; vorzugsweise ≦ 0,5 g | Vorzugsweise < 1g, insbesondere ≦ 0,5g |
| Speichervolumen | | < SP | > CB; BS | < SP |
| Mechanischer Druckwiderstand | z. B. Schiefer Kompressometer | < SP | > CB; BS | < SP |
| CB; Sperrschicht; SP: Flüssigkeitsverteilungs- und Speicherungslage; BS: Unterlage | | | | |

Eine Gewichtung der einzelnen Eigenschaften, beispielsweise für einen Damenhygieneartikel, insbesondere für eine Damenbinde, gibt die folgende Tabelle 4 wieder. Für andere Anwendungen und Einsatzgebiete kann die Gewichtung wiederum anders gestaltet sein.

**Tabelle 4**

| Gewichtung | Eigenschaft |
|---|---|
| 1 | Luftdurchlässigkeit, Wassersäule, Durchnässung |
| 2 | Druckwiderstand, Speichervolumen |
| 3 | Porengröße, Flächengewicht |

Vorzugsweise haben die verwendeten Fasern beziehungsweise Filamente für die Flüssigkeitsverteilungs- und Speicherungslage eine Fasergröße, die abhängig vom verwendeten Material ist. Beispiele hierzu gehen aus der folgenden Tabelle 5 hervor.

**Tabelle 5**

| Material/Durchmesser | 10 µm | 35 µm | 38 µm |
|---|---|---|---|
| PP | 0, 7 dtex | 8,7 dtex | 10,2 dtex |
| PET Polyester | 1,1 dtex | 13,2 dtex | 15,5 dtex |

Diese Beispiele gelten insbesondere für Filamente wie auch für Stapelfasern.

Eine weiter Ausgestaltung sieht vor, dass als Unterlage kein Film verwendet wird, sondern vielmehr ein Vliesmaterial. Die Unterlage ist daher filmfrei. Das Vliesmaterial weist vorzugsweise eine Wassersäule auf, die größer als 25 cm ist, insbesondere größer als 35 cm und vorzugsweise über 40 cm ist. Beispielsweise wird dafür ein SMS-Material eingesetzt. Je nach SMS-Materialgewicht besteht die Möglichkeit, die Wassersäule auch größer 60 cm einzustellen. So lässt gemäß einer Ausführung eine Windel wie auch eine Binde herstellen, die jeweils filmfrei sind. Beispielsweise weist ein derartiges Produkt eine der Haut entgegengewandte Oberlage, nachfolgend eine sogenannte Aquisition-Lage, anschließend den Kern, nachfolgend eine Sperrlage, an die sich eine Flüssigkeitsverteilungslage anschließt und schließlich eine filmfreie Unterlage als Aufbau auf.

Die Flüssigkeitsverteilungslage hat eine Oberflächenspannung, die nicht größer als 54 mN/m ist. Bei Verwendung eines Polyolefins und/oder PET als Faser- bzw. Filamentmaterial ist die Oberflächenspannung vorzugsweise nicht größer als 38 mN/m. Die Flüssigkeitsverteilungslage kann weiterhin zumindest ein oder mehrere Additive aufweisen, welche die Oberflächenspannung der Flüssigkeitsverteilungslage absenken. Vorzugsweise wird die Oberflächenspannung zumindest teilweise auf und/oder in der Flüssigkeitsverteilungslage auf unter 32 mN/m, vorzugsweise weniger als 30 mN/m abgesenkt. Als Additiv sind beispielsweise Siloxane, insbesondere Polydimethylsiloxane, Metallseifen und/oder flourhaltige Verbindungen einsetzbar. Die Oberflächenspannung wird in Anlehnung an IST 80.7-95 und ASTM D2578-94 nach Testmethode CM-320-A der Anmelderin bestimmt. Neben einer Absenkung durch Verwendung von zumindest einem Additiv besteht die Möglichkeit, über einen Auftrag auf die Flüssigkeitsverteilungslage die Oberflächenspannung einzustellen.

Die verwendeten Fasern für die Flüssigkeitsverteilungs- und Speicherungslage können weiterhin oberflächengeformt sein, beispielsweise einen sternförmigen oder ansonsten polygonen Querschnitt aufweisen. Die Fasern können hohl sein, kardiert, gekräuselt, aus zwei oder mehr Materialien bestehend, beispielsweise in Form eines Kerns, umhüllt von einem anderen Material, wobei beide Materialien unterschiedliche Eigenschaften aufweisen, beispielsweise in Form unterschiedlicher Dehnung, Oberflächenfestigkeit, Schmelztemperatur und anderem. Weiterhin können die Fasern fibriliert sein, eine klebrige Oberfläche besitzen, und/oder auch abbaubar sein, insbesondere biologisch abbaubar.

Eine Bindung bzw. Verfestigung der verwendeten Fasern erfolgt beispielsweise mittels einer Punktverfestigung, einer Druckverfestigung, durch Imprägnierung, mittels Bindefasern, durch Sprühverfestigung, mittels Verklebung oder anderen adhäsiven Stoffen, durch Druck und/oder Wärmeaufbringung, mittels Dampf, Wasser und/oder durch Luft, durch Vernadeln sowie durch andere Möglichkeiten. Die Bindung bzw. Verfestigung kann gemäß eines Musters erfolgen und/oder auch unregelmäßig sein.

Weitere vorteilhafte Ausgestaltungen und Weiterbildungen sind in der nachfolgenden Zeichnung ausgeführt. Dabei zeigen:
- Figur 1: eine schematische Ansicht eines Ausschnittes aus einem Wegwerfartikel mit einer Flüssigkeitsverteilungs- und Speicherungslage,
- Figur 2: den Ausschnitt aus Figur 1, wobei ein Druck wirkt,
- Figur 3: einen weiteren Ausschnitt von einem zweiten Wegwerfartikel,
- Figur 4: eine Flüssigkeitsverteilungs- und Speicherungslage in schematischer Ansicht,
- Figur 5: einen schematischen Aufbau eines Durchnässungstests,
- Figur 6 nach Figur 5,: eine schematische Ansicht der weiteren Durchführung des Tests

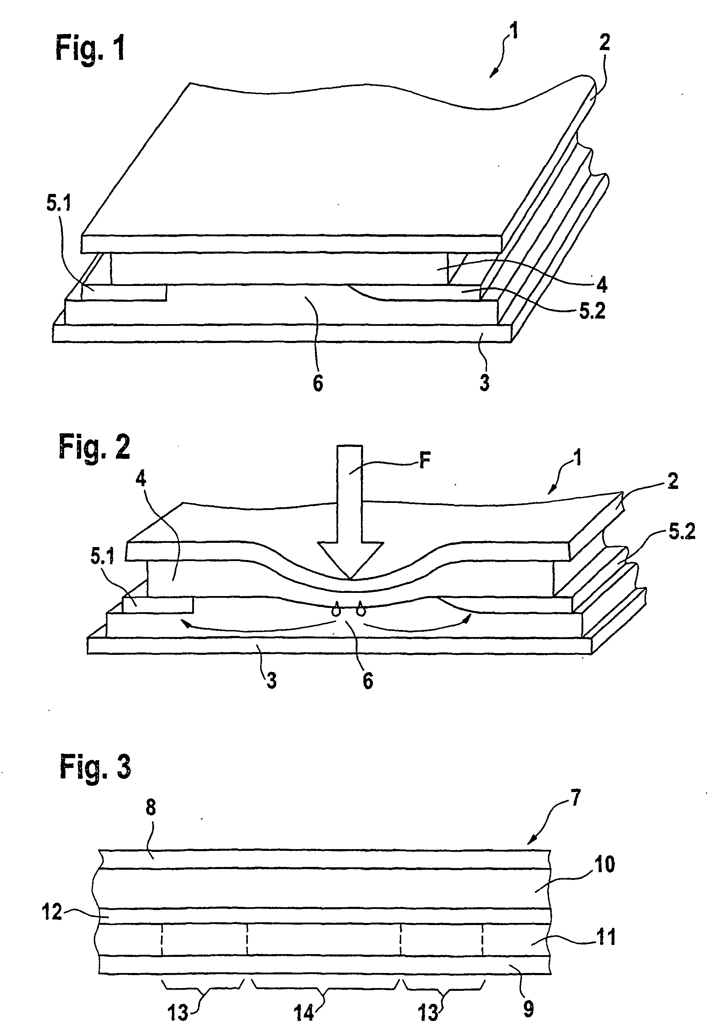

Figur 1 zeigt einen Ausschnitt aus einem Wegwerfprodukt 1 mit einer ersten Oberlage 2, einer ersten Unterlage 3, einem ersten Kern 4, einer ersten Sperrlage 5.1, einer zweiten Sperrlage 5.2 und einer ersten Flüssigkeitsverteilungs- und Speicherungslage 6. So wie in Fig. 1 dargestellt, sind die erste Sperrlage 5.1 wie auch die zweite Sperrlage 5.2 separat von der ersten Unterlage 3 angeordnet. Sie sind als Streifen angeordnet, die über den Kern 4 seitlich herausragen. Eine Geometrie der ersten Sperrlage 5.1 ist derart, dass eine in etwa flache, zu allen Seiten hin gleiche Lage verwendet wird. Die zweite Sperrlage 5.2 dagegen zeigt eine andere Ausgestaltung. Die zweite Sperrlage 5.2 hat zumindest auf einer Seite eine gebogene Oberfläche, beispielsweise durch weniger Material oder Verdichtung des dortigen Materials. Auf diese Weise wird zum Beispiel ein hydrostatischer Widerstand eingestellt werden. Neben seitlich angeordneten Sperrlagen 5 können zumindest eine oder mehr Sperrlagen auch weiter innen angeordnet sein, sich beispielsweise überlappen. So wie dargestellt, ragt auch die erste Flüssigkeitsverteilungslage 6 über den Kern 4 wie über die Sperrlagen 5 zumindest an zwei Seiten hinaus. Gemäß einer nicht dargestellten Weiterbildung ragt die Flüssigkeitsverteilungslage an allen Seiten über den Kern 4 über. Es besteht ebenfalls die Möglichkeit, dass die erste Sperrlage 5.1 und/oder zweite Sperrlage 5.2 in die erste Unterlage 3 integriert sind. Gemäß einer nicht näher dargestellten Weiterbildung ist eine zusätzliche Unterlage neben der ersten Unterlage 3 vorhanden, zwischen die die Sperrlage 5 angeordnet wird.

Figur 2 zeigt die schematische Ansicht des Ausschnitts des Wegwerfproduktes 1 aus Figur 1. Bei Aufbringen einer Druckkraft F, angedeutet mit einem Pfeil, auf die Oberlage 2 und/oder die Unterlage 3 verformt sich das Wegwerfprodukt 1. Durch den Aufbau der Flüssigkeitsverteilungslage 6 fließt bei Übersteigen eines Druckgrenzwertes ein Teil der im Kern 4 enthaltenen Flüssigkeit in die Flüssigkeitsverteilungslage 6 und wird dort seitlich verteilt. Dieses ist durch die Pfeile an zwei Tropfen Flüssigkeit angedeutet. Damit wird verhindert, dass bei Druckausübung die Sperrlage 5 bzw. Unterlage 3 von der Flüssigkeit durchdrungen werden, so dass die Flüssigkeit ausströmt. Weiterhin ist die Flüssigkeitsverteilungslage 6 in der Lage, den Kern 4 von der Unterlage 3 zu entkoppeln. Gemäß dieser Weiterbildung entkoppelt die Flüssigkeitsverteilungslage 6 den Kern 4 von der Unterlage 3. Ein lokal wirkender Spitzendruck, der beispielsweise schlagartig wirkt, drückt die aus dem Kern 4 austretende Flüssigkeit quasi nur in Richtung der Unterlage 3. Dort tritt die Flüssigkeit ein und eventuell sogar an der gegenüberliegenden Seite aus, ohne seitlich ausweichen zu können. Auf diese Weise entsteht eine vom Kern 4 bis zur Unterlage 3 reichende, durchgehende Nassbrücke. Bei Druckentlastung entkoppelt die Flüssigkeitsverteilungslage 6 diese eventuell entstandene Nassbrücke von Kern 4 und Unterlage 3, indem das durchgehende Flüssigkeitsband unterbrochen wird. Dadurch gelingt es, dass ein Nachströmen von Flüssigkeit, beispielsweise durch Kapillarwirkung oder einem sich ausbildenden Unterdruck, durch die Unterlage 3 verhindert wird. Statt dessen wird die Flüssigkeit in der Flüssigkeitsverteilungslage 6 aufgenommen und gespeichert.

Figur 3 zeigt einen weiteren Ausschnitt eines zweiten Wegwerfproduktes 7. Dieses hat eine zweite Oberlage 8, eine zweite Unterlage 9, einen zweiten Kern 10, eine zweite Flüssigkeitsverteilungs- und Speicherungslage 11 und eine optional zwischen der zweiten Flüssigkeitsverteilungslage 11 und dem zweiten Kern 10 angeordnete Sperrschicht 12. Die zweite Flüssigkeitsverteilungslage 11 weist in einem ersten Gebiet 13 bevorzugter Druckbeaufschlagung einen höheren hydrostatischen Widerstand auf gegenüber einem zweiten, benachbarten Gebiet 14 der zweiten Flüssigkeitsverteilungslage 11. Das zweite Gebiet 14 wird zu zwei Seiten von jeweils einem ersten Gebiet 13 begrenzt, wie dieses beispielsweise bei einem Wegwerfprodukt der Fall sein kann, das an eine menschliche Beckenform angepaßt ist. Das erste Gebiet 13, das zweite Gebiet 14 wie auch die zweite Flüssigkeitsverteilungslage 11 selbst können im Wegwerfprodukt streifenförmig oder auch durchgehend angeordnet sein, beispielsweise als Abschluss für Extremitäten, als eine Verstärkung, als Polsterung oder ähnliches. Die zweite Flüssigkeitsverteilungs- und Speicherungslage 11 weist vorzugsweise Gebiete mit unterschiedlich hohem hydrostatischem Widerstand auf. Dieses ist auch beim zweiten Kern 10 möglich. Beispielsweise kann ein Gebiet hohen hydrostatischen Widerstandes des zweiten Kerns 10 an einem Gebiet geringeren hydrostatischen Widerstandes der zweiten Flüssigkeitsverteilungslage 11 anliegen und umgekehrt.

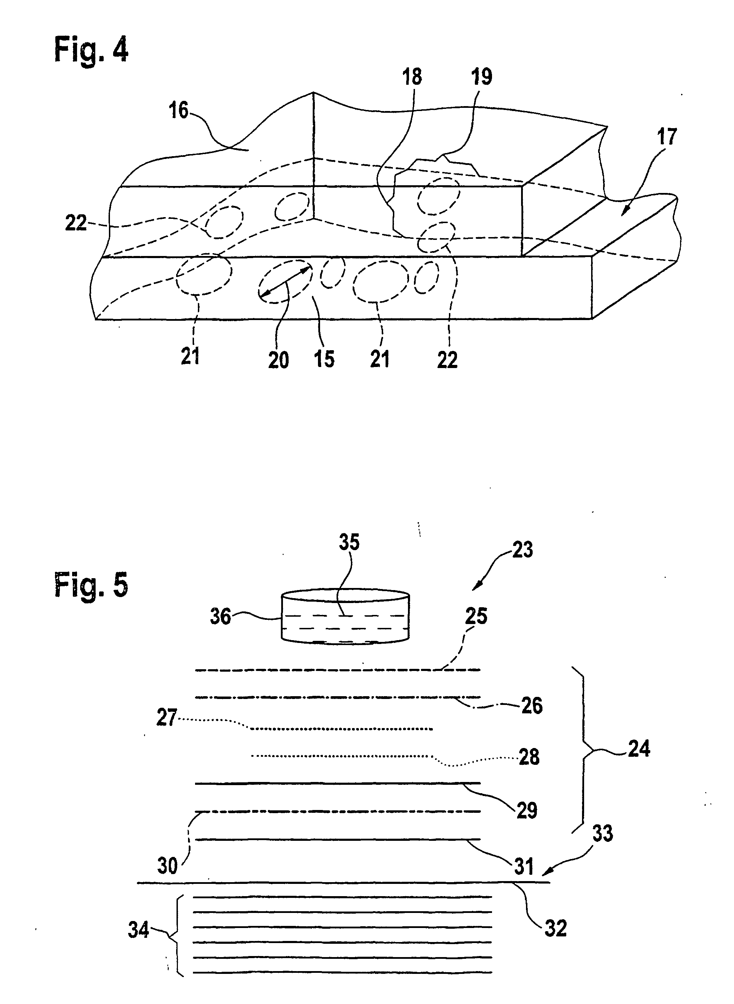

Figur 4 zeigt eine dritte Flüssigkeitsverteilungslage 15. Diese ragt seitlich über einen dritten Kern 16 hinaus. Ein herausragendes Ende 17 der dritten Flüssigkeitsverteilungslage 15 kann um den dritten Kern 16 herumgeschlungen werden, so dass seitlich aus dem dritten Kern 16 keine Flüssigkeit austreten kann, ohne nicht durch die dritte Flüssigkeitsverteilungslage 15 aufgenommen zu werden. Die dritte Flüssigkeitsverteilungslage 15 weist einen Dickenabschnitt 18 auf, der einen hydrostatischen Widerstand aufweist, der größer ist als ein am Dickenabschnitt 18 direkt benachbarter Längsabschnitt 19 der dritten Flüssigkeitsverteilungslage 15. Der Widerstand wird beispielsweise über Poren unterschiedlichen Porendurchmessers 20 eingestellt. Der Porendurchmesser 20 ermittelt sich dabei aus der Wurzel aus der Querschnittsfläche der Pore geteilt durch π. Dieses ermöglicht auch einen Vergleich unterschiedlich ausgeformter Poren. Die dritte Flüssigkeitsverteilungslage 15 hat daher zumindest eine erste Porengröße 21 und eine zweite Porengröße 22, die sich voneinander unterscheiden. Darüber ist beispielsweise ein Kapillareffekt innerhalb der dritten Flüssigkeitsverteilungslage 15 einstellbar. Auch über die Anordnung der Poren lässt sich der hydrostatische Widerstand innerhalb der Flüssigkeitsverteilungslage 15 einstellen. Die Poren können in horizontaler oder vertikaler Richtung oder entsprechend geneigt ihren größten Querschnitt aufweisen. Eine Variierung des Durchflusswiderstandes ist einerseits über die Anzahl von Poren wie aber auch über die Größe der Poren und der damit verbundenen Zwischenräume möglich. Andere Möglichkeiten bietet eine geeignete Auswahl von Filamentdurchmessern, unterschiedlichen Materialien, Zugabe von Substanzen oder anderes.

Fig. 5 zeigt einen schematischen Aufbau 23 eines Durchnässungstests ("wetthrough"). Gemäß dieses dargestellten Tests wird ein mehrlagiges Gebilde 24 aufgebaut, welches eine dritte Oberlage 25, eine sich daran anschließende vierte Oberlage 26, eine Lage Adsorptions-Distributions-Lage 27 (ADL), einen vierten Kern 28, eine zweite Sperrschicht 29, eine vierte Flüssigkeitsverteilungs- und Speicherungslage 30 und eine dritte Unterlage 31 hat. Dieses mehrlagige Gebilde 24 wird auf einen Baumwollstoff 32 aufgelegt, wobei eine glatte Seite 33 des Baumwollstoffes 32 in Richtung des mehrlagigen Gebildes 24 angeordnet ist. Sofern ,ein SM-Material im mehrlagigen Gebilde 24 vorhanden ist, so zeigt die Meltblown-Seite in die gleiche Richtung wie die glatte Seite 33. Wird ein Laminat mit einem Film eingesetzt, zeigt die Filmseite in die Richtung der glatten Seite 33 bzw. der Meltblown-Seite. Unterhalb des Baumwollstoffes 32 befinden sich sechs Lagen Filterpapier 34. Die dritte Oberlage 25, die vierte Oberlage 26, der Baumwollstoff 32 sowie das Filterpapier 34 sind quadratisch geformt und haben eine Abmessung von jeweils 10 x 10 cm. Die zweite Sperrschicht 29, die vierte Flüssigkeitsverteilungs- und Speicherungslage 30 sowie die dritte Unterlage 31 haben ebenfalls eine quadratische Form mit den Längenabmaßen 12,5 x 12,5 cm. Der dritte Kern 28 sowie die ADL-Lage 27 sind rechteckig geformt und haben die Abmaße 10 x 5 cm. Das Filterpapier 34 ist ein Filterpapier von Hollingsworth and Vose, Analytical and Industrial Filterpaper ERT FF3 "Strike through/Wetback". Der Baumwollstoff 32 hat ein Flächengewicht von 12,5 g pro Quadratmeter.

Der in Fig. 5 dargestellte Test wird wie folgt ausgeführt: Zuerst wird das Filterpapier 34 sowie der Baumwollstoff 32 eingewogen. Anschließend wird eine Testflüssigkeit 35, insbesondere synthetisches Blut, in einen Ring 36 eingegossen. Der Ring 36 hat einen inneren Durchmesser von 3 cm. Eingegossen werden 10 ml der Testflüssigkeit 35. Anschließend wird drei Minuten abgewartet und sodann der Ring 36 von der Oberfläche abgenommen. Die Testflüssigkeit, synthetisch hergestellte blutähnliche Flüssigkeit, wird nach der Prüfmethode CM-117-B der Corovin GmbH hergestellt.

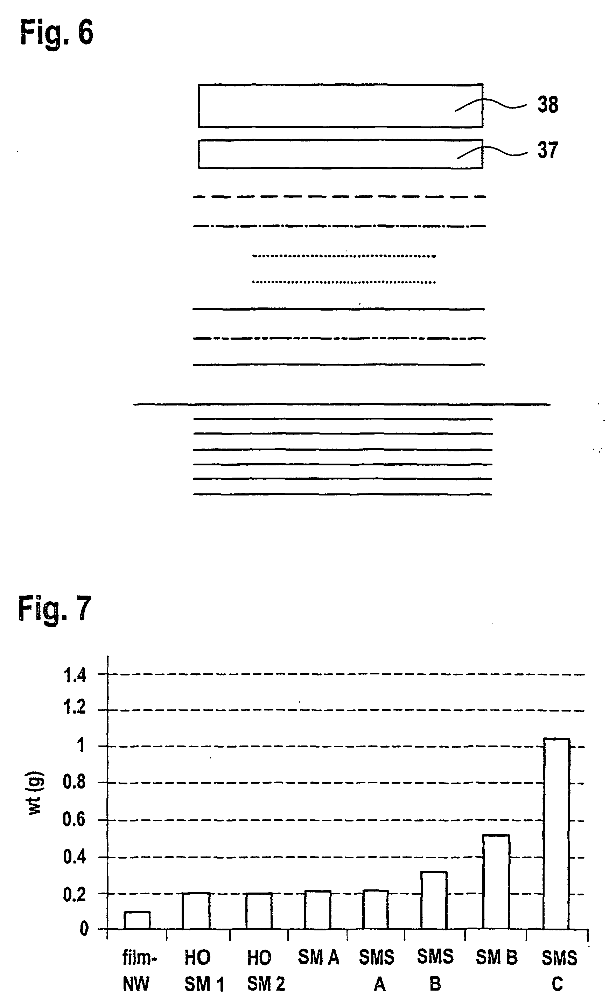

Der weitere Ablauf des Tests geht aus Fig. 6 hervor. Nachdem der Ring 36 von der dritten Oberlage 25 abgenommen wurde, wird eine Platte 37 aufgelegt. Die Platte 37 ist beispielsweise durchscheinend, vorzugsweise eine Plexiglasplatte. Anschließend wird auf die Platte 37 ein Gewicht 38 aufgelegt, das 3,5 kg wiegt. Das Gewicht 38 drückt das mehrlagige Gebilde 24 zusammen. Nach 10 Minuten wird das Gewicht 38 von dem mehrlagigen Gebilde 24 weggenommen. Die aus dem mehrlagigen Gebilde 24 ausgetretene Flüssigkeit ("wet-through") wird aus dem Gewicht von Filterpapier 34 und Baumwollstoff 32 vor dem Auftragen der Flüssigkeit und nach Durchführung des Tests berechnet. Weiterhin wird eine Fleckenbildung auf dem Baumwollstoff 32 untersucht. Dabei können beispielsweise eine Fleckenlänge und Fleckenbreite auf der glatten Seite 33 gemessen werden, um daraus wird eine gemittelte Fläche zu ermitteln. Überschreitet dieser gemittelte Flächenwert einen vorgebbaren Grenzwert, ist das getestete mehrlagige Gebilde 24 nicht für ein Wegwerfprodukt mit den geforderten Ansprüchen bezüglich Nichtverschmutzung und Nichtdurchlässigkeit von Flüssigkeit geeignet. Gemäß einer weiteren Ausgestaltung wird von der Fleckenlänge auf die Eignung des Gebildes für eine bestimmte Anwendung geschlossen.

Aus den folgenden Figuren 7 bis 12 gehen Werte zur Durchnässung und zur Fleckbildung von verschiedenen Materialien und unterschiedlichen Aufbauten der Testobjekte hervor. Die in den einzelnen Figuren benannten Materialien gehen aus der folgenden Tabelle 6 hervor.

**Tabelle 6**

| Material | Funktion als | Aufbau | Eigenschaft |
|---|---|---|---|
| Film-Vlies | BS (oder CB) | Laminat aus mikroporöser Film und Vlies | Hohe Barrierewirkung |
| HO SM1 | BS (oder CB) | Vliesstoff: Offline Spinnvlies -schmelzgeblasenes Vlies, 28 g/m², inkl. Additive 1 | Hohe Barrierewirkung |
| HO SM2 | BS (oder CB) | Vliesstoff: Offline Spinnvlies -schmelzgeblasenes Vlies, 28 g/m², inkl. Additive 2 | Hohe Barrierewirkung |
| SM A | BS (oder CB) | Vliesstoff: Offline Spinnvlies -schmelzgeblasenes Vlies, 28 g/m², | Hohe Barrierewirkung |
| | | | |
| SMS A | (BS oder) CB | Vliesstoff: Inline Spinnvlies -schmelzgeblasenes Vlies Spinnvlies (S:~13 µm) 24 g/m² | Mittlere bis niedrige - Barrierewirkung |
| SMS B | (BS oder) CB | Vliesstoff: Inline Spinnvlies -schmelzgeblasenes Vlies -Spinnvlies (S:~17 µm) 24 g/m² | Mittlere bis niedrige Barrierewirkung |
| SMS C | (BS oder) CB | Vliesstoff: Inline Spinnvlies -schmelzgeblasenes Vlies -Spinnvlies (S:~17 µm) 16 g/m² | Mittlere bis niedrige Barrierewirkung |
| SM B | (BS oder) CB | Vliesstoff: Inline Spinnvlies -schmelzgeblasenes Vlies -Spinnvlies 25 g/m² | Mittlere bis niedrige Barrierewirkung |
| | | | |
| HO S A | SP | Spinnvlies, 19 µm, 20 g/m² | |
| HO S B | SP | Spinnvlies, 24 µm, 20 g/m² | |
| Loft AT | SP | Hochvoluminöses, heißluftverfestigtes Vlies, 50 g/m² | Leervolumen |
| Loft Cal. | SP | Voluminöses Kalanderverfestigtes Vlies, 30 g/m², Oberflächenbehandelt | Leervolumen und Wasserabstoßfähig |
| Additive: aus der Gruppe der Flourverbindungen, Polydimethylsiloxane, Metallseifen oder andere | | | |

Aus den Testergebnissen ist abzuleiten, dass durch die Verwendung einer Flüssigkeitsverteilungs- und Speicherungslage beispielsweise eine schwächere Sperrschicht am Kern verwendet werden kann. Die Flüssigkeitsverteilungslage reduziert das Austreten von Flüssigkeit und eine Fleckenbildung deutlich. Wird an die Flüssigkeitsverteilungslage eine luftdurchlässige Unterlage hinzugefügt, erfolgt eine weitere Reduzierung. Die Testmethode "Durchnässung und Fleckenbildung" (Wet through) detektiert den Durchlass von Feuchtigkeit als Wasserdampf (WDD) und ermöglicht das Testen unterschiedlicher Materialkominationen. Der Test ermöglicht die Güte einer Flüssigkeitsverteilungslage im Zusammenspiel mit weiteren Lagen wie einer Sperrlage und/oder einer Unterlage einzustufen.

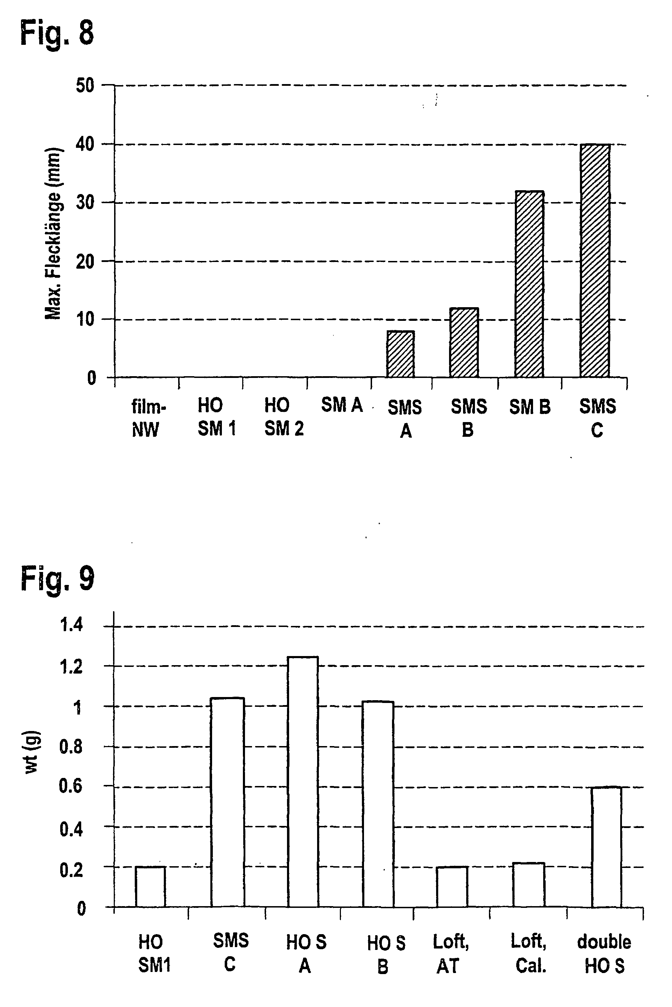

Figur 7 und Figur 8 zeigen das Verhalten von Barriere-Materialien, die jeweils einzeln getestet wurden, um ihre unterschiedlichen Güten beziehungsweise Durchlässigkeiten zu ermitteln. Dazu wurden die Barriere-Materialien unter einem Kern angeordnet und anschließen der oben beschriebene "Durchnässungs- und Fleckenbildungstest" durchgeführt. Von dem Laminat Film-Vliesstoff bis zum Laminat SM A weisen die Materialien eine hohe Barrierewirkung auf. Daher ist die Durchnässung gering. Eine Fleckenbildung war nicht nachweisbar. Von Laminat SMS A bis zum Laminat SMS C dagegen war nur eine geringe bis mittlere Barrierewirkung festzustellen. Einerseits traten höhere Durchnässungswerte auf. Andererseits waren deutliche Flecken zu erkennen.

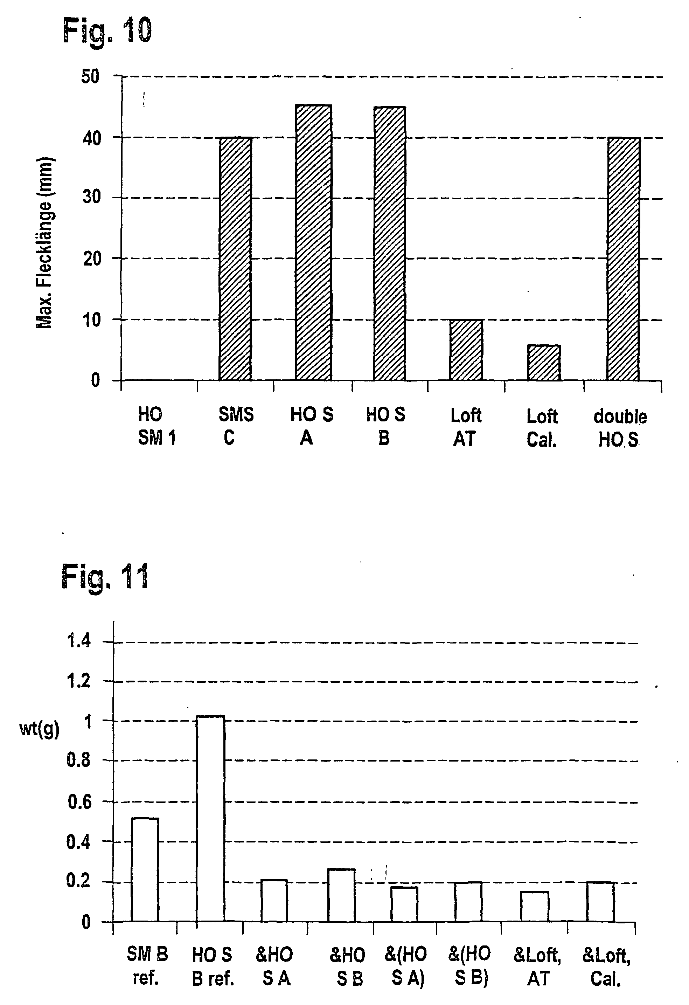

Figur 9 und Figur 10 zeigen die erzielten Werte an Durchnässung wie an einer Fleckenbildung für eine Anordnung einer Flüssigkeitsverteifungs- und Speicherungslage direkt unter einem Kern. Als Referenzmuster ist HO SM 1 mitgetestet worden. Die Werte geben Aufschluß über eine Offenheit der Struktur der jeweils getesteten Materialien wie auch über das in den Materialien enthaltene freie Volumen. Zu erkennen ist der deutliche Strukturunterschied beispielsweise zwischen dem Referenzmuster sowie SMS C, HO S A und HO S B. Die Durchnässung ist teilweise um das Fünfache größer. Ebenfalls getestet wurde eine doppelte Lage HO S, die zwar nur eine dreifach größere Durchnässung aufwies. Die Fleckbildung dagegen unterschied sich kaum von SMS C, HO S A und HO S B. Eine sehr geringe Durchnässung wiesen Loft AT und Loft Cal. auf, wobei die Fleckenbildung bei Loft Cal. gegenüber der von Loft AT geringer ausfiel. Die Fleckenbildung war jedoch so gering, dass derartige Materialien beispielsweise direkt in Verbindung mit einem Kern ohne Barrierematerial dazwischen in einem Hygieneprodukt einsetzbar sind.

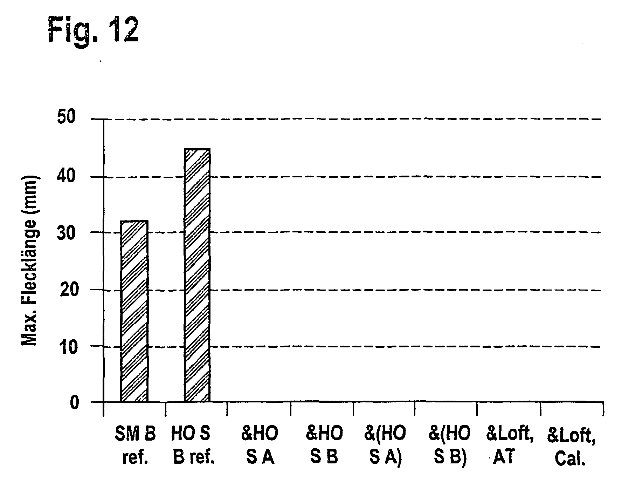

Figur 11 und Figur 12 zeigen die Testergebnisse für einen mehrlagigen Aufbau mit einem Kern, an den sich eine Sperrlage aus SM B anschloß. An diese wiederum wurden verschiedene Flüssigkeitsverteilungs- und Speicherungslagen angelagert. Festzustellen war ein verminderter Flüssigkeitsaustritt aus dem Kern aufgrund der Barrierelage. Die an die Barrierelage angrenzende Flüssigkeitsverteilungslage war in der Lage, die durchtretende Flüssigkeit aufzunehmen, zu verteilen und zu speichern. Unter einem bestimmten Messwert der Durchnässung wurde keine Fleckbildung mehr beobachtet. Der zu beobachtende Feuchtigkeitsaustritt basierte auf Wasserdampf. In dem in Figuren 11 und 12 dargestellten Beispiel lag die Grenze bei 0,2 Gramm Durchnässung. Zum Vergleich sind zwei Referenzmaterialien angegeben, die ebenfalls getestet wurden. Diese erreichten jedoch nicht die Aufnahme und Verteilung der Flüssigkeit wie bei den anderen. Daher wurde auch eine deutliche Fleckenbildung bei den Referenzmaterialien festgestellt.

Mögliche und vorteilhafte Zusammenstellungen verschiedener Materialien aufgrund obiger Ergebnisse lassen sich aus der folgenden Tabelle 7 entnehmen.

**Tabelle 7**

| Typ | Material |
|---|---|
| 1 | CB mit hoher Barrierewirkung + leichter SP + BS mit mittlerer Barrierewirkung |
| 2 | CB mit mittlerer Barrierewirkung + mittlere SP + BS mittlerer Barrierewirkung |
| 3 | CB mittlerer Barrierewirkung +schwere/dicke SP + BS leichter Barrierewirkung |
| 4 | CB leichter Barrierewirkung + schwere/dicke SP+ BS mittlerer Barrierewirkung |
| 5 | Schwere/dicke SP + BS mit hoher Barrierewirkung |
| 6 | Schwere/dicke SP + BS mit mittlerer Barrierewirkung |
| CB Sperrlage (Core barrier); SP Flüssigkeitsverteilungs- und Speicherungslage; BS Unterlage (Backsheet) | |

Auf das Verhalten eines gewählten Aufbaus wirken verschiedene Einflussparameter, die entsprechend angepasst werden können für unterschiedliche Anwendungen. Beeinflusst wird der Aufbau beispielsweise über die Anzahl und Art und Weise der einander überlappenden Lagen, der Lagenmaterialien, der Fasergröße, der Porengröße, der Dicke, das Flächengewicht, das Leervolumen, die Faserorientierung, die Luftdurchlässigkeit, die Oberflächenchemie und/oder andere Einflüsse.

Beispiele für die einzelnen Einflussparameter können wie folgt sein:
a) einander überlappende Lagen
   Die Flüssigkeitsverteilungslage ist kleiner, gleich oder größer auf mindestens einer Seite als eine andere Lage.
b) Fasergröße
   Der Faserdurchmesser kann unterschiedlich lang gewählt sein, zum Beispiel für Stapelfasern (endlich lang) oder Filamente (endlos lang), für die Sperrlage, die Unterlage oder einen Film.
c) Dicke
   Für eine Flüssigkeitsverteilungslage beispielsweise beträgt die Dicke vorzugsweise mehr als 50 µm, insbesondere bis zu 3000µm:
d) Leervolumen
   Die Flüssigkeitsverteilungslage ist ein Gebilde aus Faser mit vorzugsweise geringer Dichte, aber großer Dicke. Dadurch erhält die Flüssigkeitsverteilungslage ein großes freies Volumen, das Flüssigkeit aufnehmen kann.
e) Faserorientierung
   Faserorientierung im Vlies, das bedeutet, das Längs/Querverhältnis, welches messbar als mechanische Festigkeit längs und quer zur Produktionsrichtung ist. Beispielsweise wird folgendes Verhältnis Längs (MD): Quer (CD) aus dem nachfolgenden Bereich eingestellt: 0,1:1 bis zu 30:1, vorzugsweise 1:1 bis 10:1 und insbesondere 1:1 bis 5:1.
f) Luftdurchlässigkeit
   Dieses entspricht einem Volumenstron an Luft, der durch das Gebilde strömen kann. Die Luftdurchlässigkeit bedeutet dabei mehr als nur Atmungsaktivität (Wasserdampfdurchlässigkeit WDD). Die Luftdurchlässigkeit der Flüssigkeitsverteilungslage sollte beispielsweise über 10 m³/m²/min betragen.
   Weitere Einflussparameter sind beispielsweise in Tabelle 3 enthalten.

Die Flüssigkeitsverteilungslage beziehungsweise das Wegwerfprodukt sind beispielsweise in Hygieneprodukten wie Windeln, insbesondere für Neugeborene in den ersten vier Wochen, Inkontinenzartikel, Binden, Sanitärprodukten, Reinigungsprodukten, Verbandsauflagen, Wundkompressen, Krankenhausartikeln wie Abdeckungen für Operationstische, Betten, Bettenauflagen und ähnlichem einsetzbar ebenso wie im Lebensmittelbereich, beispielsweise bei Lebensmittelverpackungen. Gemäß einer Weiterbildung ist das Wegwerfprodukt auch mehrfach verwendbar. Auch kann ein Produkt die Flüssigkeitsverteilungslage und den Kern nur in bestimmten Abschnitten aufweisen, beispielsweise bei Durchgängen oder Umfassungen, die möglichst flüssigkeitsdicht sein sollen.

### Bezugszeichenliste

- 1: Ausschnitt aus Wegwerfprodukt
- 2: Erste Oberlage
- 3: Erste Unterlage
- 4: Erste Kern
- 5: Sperrlage
- 6: Erste Flüssigkeitsverteilungs- und Speicherungslage
- 7: Ausschnitt aus einem zweiten Wegwerfprodukt
- 8: Zweite Oberlage
- 9: Zweite Unterlage
- 10: Zweiter Kern
- 11: Zweite Flüssigkeitsverteilungs- und Speicherungslage
- 12: Sperrschicht
- 13: Erstes Gebiet bevorzugter Druckbeaufschlagung
- 14: Zweites Gebiet
- 15: Dritte Flüssigkeitsverteilungs- und Speicherungslage
- 16: Dritter Kern
- 17: Hinausragendes Ende
- 18: Dickenabschnitt
- 19: Längsabschnitt
- 20: Porendurchmesser
- 21: Erste Porengröße
- 22: Zweite Porengröße
- 23: Testaufbau
- 24: Mehrlagiges Gebilde
- 25: Dritte Oberlage
- 26: Vierte Oberlage
- 27: ADL
- 28: vierter Kern
- 29: Zweite Sperrschicht
- 30: Vierte Flüssigkeitsverteilungs- und Speicherungslage
- 31: Dritte Unterlage
- 32: Baumwollstoff
- 33: Glatte Seite
- 34: Filterpapier
- 35: Testflüssigkeit
- 36: Ring
- 37: Platte
- 38: Gewicht

## Patentansprüche

1. Flüssigkeitsaufnehmendes Wegwerfprodukt (1; 2), insbesondere Hygieneprodukt, mit einer Oberlage (2; 8; 25) und einem flüssigkeitsaufnehmenden und speichernden Kern (4; 10; 16; 28), wobei dem Kern (4; 10; 16; 28) eine Flüssigkeitsverteilungs- und Speicherungslage (6; 11; 15; 30) nachgeordnet ist, um Flüssigkeit aufzunehmen, die aus dem flüssigkeitsaufnehmenden Kern bei Druckausübung (4; 10; 16; 28) austritt,
**dadurch gekennzeichnet, dass** die Flüssigkeitsverteilungslage (6; 11; 15; 30) ein Vlies aufweist mit einem größeren mittleren Porendurchmesser (20) als eine zur Flüssigkeitsverteilungslage (6; 11; 15; 30) angrenzende Lage und mit einer Oberflächenspannung, die gleich oder kleiner 54 mN/m ist, wobei in der Flüssigkeitsverteilungslage (6; 11; 15; 30) bei Druckausübung ein geringerer Strömungswiderstand bezüglich einer Verteilung einer Flüssigkeit innerhalb der Flüssigkeitsverteilungslage (6; 11; 15; 30) herrscht gegenüber einem Strömungswiderstand zum Austreten der Flüssigkeit aus der Flüssigkeitsverteilungslage (6; 11; 15; 30).

2. Wegwerfprodukt (1; 2) nach Anspruch 1,
**dadurch gekennzeichnet, dass** eine an die Flüssigkeitsverteilungslage (6; 11; 15; 30) angrenzende Lage einen höheren hydrostatischen Druckwiderstand aufweist als die Flüssigkeitsverteilungslage (6; 11; 15; 30).

3. Wegwerfprodukt (1; 2) nach einem der vorhergehenden Ansprüche,
**dadurch gekennzeichnet, dass** die Flüssigkeitsverteilungslage (6; 11; 15; 30) einen uneinheitlichen Aufbau aufweist.

4. Wegwerfprodukt (1; 2) nach einem der vorhergehenden Ansprüche,
**dadurch gekennzeichnet, dass** zumindest die Flüssigkeitsverteilungslage (6; 11; 15; 30) einen Saugeffekt, insbesondere einen Kapillareffekt, aufweist.

5. Wegwerfprodukt nach einem der vorhergehenden Ansprüche,
**dadurch gekennzeichnet, dass** der Kern einen Saugeffekt aufweist, um Flüssigkeit, die sich in der Flüssigkeitsverteilungslage befindet, wieder zurücksaugen zu können.

6. Wegwerfprodukt (1; 2) nach einem der vorhergehenden Ansprüche,
**dadurch gekennzeichnet, dass** die Flüssigkeitsverteilungslage (6; 11; 15; 30) ein durchflusshemmendes Mittel aufweist, dass in Kontakt mit einer Flüssigkeit reagiert.

7. Wegwerfprodukt (1; 2) nach einem der vorhergehenden Ansprüche,
**dadurch gekennzeichnet, dass** die Flüssigkeitsverteilungslage (6; 11; 15; 30) zumindest seitlich über den Kern (4; 10; 16; 28) hinausragt.

8. Wegwerfprodukt (1; 2) nach einem der vorhergehenden Ansprüche,
**dadurch gekennzeichnet, dass** zwischen der Flüssigkeitsverteilungslage (6; 11; 15; 30) und dem Kern (4; 10; 16; 28) eine Sperrschicht (12; 29) angeordnet ist.

9. Wegwerfprodukt nach einem der vorhergehenden Ansprüche,
**dadurch gekennzeichnet, dass** die Flüssigkeitsverteilungslage den Kern von einer Unterlage entkoppelt.

10. Wegwerfprodukt nach einem der vorhergehenden Ansprüche,
**dadurch gekennzeichnet, dass** es ein Damenhygieneartikel ist, wobei die Flüssigkeitsverteilungslage zumindest teilweise eine Außenlage des Damenhygieneartikels bildet.

11. Flüssigkeitsaufnehmendes Wegwerfprodukt (1; 2) nach Anspruch 1, mit einer Flüssigkeitsverteilungs- und Speicherungslage (6; 11; 15; 30) mit den Merkmalen nach einem der Ansprüche 1 bis 10.

12. Flüssigkeitsaufnehmendes Wegwerfprodukt (1; 2) nach Anspruch 11,
**dadurch gekennzeichnet, dass** der Strömungswiderstand in der Flüssigkeitsverteilungs- und Speicherungslage (6; 11; 15; 30) über ein Bondingmuster eingestellt ist, wobei die Flüssigkeitsverteilungs- und
Speicherungsschicht (6; 11; 15; 30) ungleichmäßig ausgestaltet ist, derart, dass das Verbindungs- oder Bondingmuster einen Bereich eingrenzt, der ohne Verbindungsstellen ausgestaltet ist und dass der zu einem Verbindungsbereich mit einem Bondingmuster angrenzenden Bereich aufgrund der Verbindungsstellen engere Poren und/oder engere Zwischenräume aufweist

## Claims

1. A fluid-absorbing, disposable product (1; 2), in particular a sanitary product, comprising a top layer (2; 8; 25) and a fluid-absorbing and fluid-storing core (4; 10; 16; 28), whereby a fluid distribution and fluid storage layer (6; 11; 15; 30) is subordinated to said core (4; 10; 16; 28) in order to absorb a fluid escaping from said fluid-absorbing core when pressure (4; 10; 16; 28) is exerted, **characterized in that** said fluid distribution layer (6; 11; 15; 30) comprises a nonwoven having a larger mean pore diameter (20) than a layer adjacent to said fluid distribution layer (6; 11; 15; 30) and having a surface tension which equals or is below 54 mN/m, whereby with respect to the distribution of a fluid within said fluid distribution layer (6; 11; 15; 30) on the exertion of pressure a lower flow resistance is present in said fluid distribution layer (6; 11; 15; 30) than the flow resistance relating to the escaping of the fluid from said fluid distribution layer (6; 11; 15; 30).

2. A disposable product (1; 2) according to claim 1, **characterized in that** a layer adjacent to said fluid distribution layer (6; 11; 15; 30) has a higher hydrostatic pressure drag than said fluid distribution layer (6; 11; 15; 30).

3. A disposable product (1; 2) according to any of the preceding claims, **characterized in that** said fluid distribution layer (6; 11; 15; 30) has an inconsistent structure.

4. A disposable product (1; 2) according to any of the preceding claims, **characterized in that** at least said fluid distribution layer (6; 11; 15; 30) has a suction effect, in particular a capillary action.

5. A disposable product according to any of the preceding claims, **characterized in that** said core has a suction effect, in order to be able to suck back fluid which is present in said fluid distribution layer.

6. A disposable product (1; 2) according to any of the preceding claims, **characterized in that** said fluid distribution layer (6; 11; 15; 30) comprises an agent, which reacts when in contact with fluid and prevents discharge from flowing through.

7. A disposable product (1; 2) according to any of the preceding claims, **characterized in that** said fluid distribution layer (6; 11; 15; 30) protrudes at least sideways from said core (4, 10; 16, 28).

8. A disposable product (1; 2) according to any of the preceding claims, **characterized in that** a barrier layer (12; 29) is arranged between said fluid distribution layer (6; 11; 15; 30) and said core (4; 10; 16; 28).

9. A disposable product according to any of the preceding claims, **characterized in that** said fluid distribution layer uncouples said core from a bottom layer.

10. A disposable product according to any of the preceding claims, **characterized in that** it is a feminine sanitary product, whereby said fluid distribution layer at least partially forms an outer layer of said feminine sanitary product.

11. A fluid-absorbing, disposable product (1; 2) according to claim 1 comprising a fluid distribution and fluid storage layer (6; 11; 15; 30) featuring the characteristics according to any of the claims 1 to 10.

12. A fluid-absorbing, disposable product (1; 2) according to claim 11, **characterized in that** the flow resistance in said fluid distribution and fluid storage layer (6; 11; 15; 30) is adjusted by means of a bonding pattern, whereby said fluid distribution and fluid storage layer (6; 11; 15; 30) features an irregular design, so that said connecting or bonding pattern encloses an area not featuring any connection points and the area bordering a connection area featuring said bonding pattern comprises smaller pores and/or smaller gaps due to said connection points.

## Revendications

1. Produit jetable (1; 2) absorbant les liquides, en particulier un produit hygiénique, qui comprend successivement une couche supérieure (2; 8; 25), une âme (4; 10; 16; 28) absorbant et stockant les liquides et une couche de répartition et de stockage des liquides (6; 11; 15; 30) qui absorbe un liquide qui sort de l'âme en cas de pression (4; 10; 16; 28) exercée sur cette dernière, **caractérisé en ce que** la couche de répartition des liquides (6; 11; 15; 30) possède un non-tissé doté d'un diamètre de pore (20) moyen plus grand qu'une couche avoisinante de la couche de répartition des liquides (6; 11; 15; 30) et ayant une tension de surface qui est égale ou inférieure à 54 mN/m, dans laquelle règne en cas de pression une résistance de courant plus faible en ce qui concerne une répartition d'un liquide à l'intérieur de la couche de répartition des liquides (6; 11; 15; 30) par rapport à la résistance de courant lors de la sortie des liquides de la couche de répartition des liquides (6; 11; 15; 30).

2. Produit jetable (1; 2) selon la revendication 1 **caractérisé en ce qu'**une couche adjacente à la couche de répartition des liquides (6; 11; 15; 30) possède une plus haute résistance de pression hydrostatique que la couche de répartition des liquides (6; 11; 15; 30).

3. Produit jetable (1; 2) selon l'une quelconque des revendications précédentes **caractérisé en ce que** la couche de répartition des liquides (6; 11; 15; 30) possède une structure hétérogène.

4. Produit jetable (1; 2) selon l'une quelconque des revendications précédentes **caractérisé en ce que** la couche de répartition des liquides (6; 11; 15; 30) présente au moins un effet aspirant, en particulier un effet capillaire.

5. Produit jetable selon l'une quelconque des revendications précédentes **caractérisé en ce que** l'âme possède un effet aspirant afin de pouvoir ré-aspirer le liquide qui se trouve dans la couche de répartition des liquides (6; 11; 15; 30).

6. Produit jetable (1; 2) selon l'une quelconque des revendications précédentes **caractérisé en ce que** la couche de répartition des liquides (6; 11; 15; 30) possède un moyen entravant le débit qui agit en contact avec un liquide.

7. Produit jetable (1; 2) selon l'une quelconque des revendications précédentes **caractérisé en ce que** la couche de répartition des liquides (6; 11; 15; 30) dépasse du moins sur le côté l'âme (4; 10; 16; 28).

8. Produit jetable (1; 2) selon l'une quelconque des revendications précédentes **caractérisé en ce qu'**une couche d'arrêt (12; 29) est disposée entre la couche de répartition des liquides et l'âme (4; 10; 16; 28).

9. Produit jetable selon l'une quelconque des revendications précédentes **caractérisé en ce que** la couche de répartition des liquides (6; 11; 15; 30) découple l'âme d'une couche inférieure.

10. Produit jetable selon l'une quelconque des revendications précédentes **caractérisé en ce qu'**il s'agit d'un produit hygiénique féminin duquel la couche de répartition des liquides constitue pour le moins en partie une couche extérieure du produit hygiénique féminin.

11. Produit jetable absorbant les liquides (1; 2) selon la revendication 1 comprenant une couche de répartition et de stockage des liquides (6; 11; 15; 30) avec les caractéristiques selon l'une quelconque des revendications 1 à 10.

12. Produit jetable absorbant les liquides selon la revendication 11 **caractérisé en ce que** la résistance de courant dans la couche de répartition et de stockage des liquides (6; 11; 15; 30) est ajustée au moyen d'un motif de consolidation par lequel la couche de répartition et de stockage des liquides (6; 11; 15; 30) est formée inégalement de manière à ce que le motif de liaison ou de consolidation délimite un domaine qui n'est pas doté de points de jonction et que le domaine avoisinant celui-ci ayant un motif de consolidation doté en raison de ces points de jonction de pores plus restreints et/ou d'espaces plus étroits.
